# FASCICULE DE BREVET EUROPEEN

(11) **EP 0 959 822 B1**
(45) Date de publication et mention de la délivrance du brevet: **19.05.2004**
(21) Numéro de dépôt: 97900650.9
(22) Date de dépôt: 15.01.1997
(51) Int. Cl.: A61F 2/40, A61F 2/46

(54) **JEU DE PROTHESES DE TETE HUMERALE**
SATZ VON HUMERUSKOPFPROTHESEN
SET OF HUMERAL HEAD PROSTHESES

(30) Priorité: 15.01.1996 FR 9600373
(43) Date de publication de la demande: 01.12.1999
(73) Titulaire: Depuy France, 69100 Villeurbanne (FR)
(72) Inventeur: DELINCE, Philippe, B-1380 Lasne (BE); DESMOINEAUX, Pierre, F-78150 Le Chesnay (FR); DUMONTIER, Philippe, F-09000 Bressoles (FR); HOUSSARD, Patrick, F-21380 Marsannay-le-Bois (FR); ROUSSEL, Charly, F-52000 Nogent (FR)
(74) Mandataire: Bernasconi, Jean
(86) Numéro de dépôt international: PCT/FR1997/000070
(87) Numéro de publication internationale: WO 1997/025943

(56) Documents cités:
- EP-A- 0 339 530
- EP-A- 0 558 203
- EP-A- 0 679 375
- WO-A-93/09733
- WO-A-94/15551
- WO-A-95/22302
- FR-A- 2 579 454
- FR-A- 2 660 857
- FR-A- 2 685 633
- FR-A- 2 703 241

## Description

La présente invention a trait à un jeu de prothèses d'épaule et, plus précisément, à un jeu de têtes humérales.

De telles prothèses sont destinées à remplacer la tête humérale et à former, à l'extrémité de l'humérus, une surface articulaire destinée à coopérer avec la glène ou une cupule d'articulation implantée dans la cavité glénoïde de l'omoplate.

Elle comporte, classiquement, la surface articulaire en forme de calotte sphérique, une queue destinée à être introduite et fixée dans le canal médullaire et un col.

Anciennement, les prothèses de ce genre était réalisée sous forme monobloc, c'est-à-dire d'un seul tenant, ce qui demandait de prévoir un très grand nombre de prothèses de dimensions différentes, compte tenu des variations des dimensions de la queue, d'une part, et de la calotte sphérique, d'autre part.

Afin de remédier à ce problème, on a déjà proposé, dans le brevet français FR-A-2 685 633, une prothèse humérale modulaire composée de trois éléments assemblables, à savoir une queue, une calotte sphérique, et une entretoise intermédiaire formant col et interposée entre la tête et la queue. Afin d'assurer l'adaptation aux différentes circonstances il était nécessaire de disposer d'un grand nombre d'entretoises de taille et d'inclinaison différentes.

On trouve également sur le marché des jeux de prothèses plus simples, constitués de trois ou quatre prothèses de taille différente se caractérisant par une calotte ayant un même diamètre et un même rayon de courbure pour toutes les prothèses du jeu, les différentes prothèses d'un jeu se distinguant les unes des autres par la hauteur du col, dont la face inférieure ou base présente un téton de centrage destiné à venir se disposer et s'immobiliser dans un trou complémentaire pratiqué dans la face de la queue contre laquelle s'applique la face inférieure du col.

Le téton peut être disposé centralement dans la prothèse, ou être excentré, ce qui permet alors, en ajustant la position angulaire de la prothèse humérale, de déporter l'axe de symétrie de la calotte sphérique de façon à reproduire le décalage, généralement en rétroversion, de la tête humérale avant intervention.

De telles prothèses sont décrites notamment dans le brevet FR-A-2 703 241, qui prévoit également d'incliner l'axe de symétrie de la tête humérale par rapport à l'axe de symétrie du téton en réalisant un col inscrit dans un dièdre.

En fait, le principal problème que cherchent à résoudre ces jeux de prothèses et, éventuellement, de pièces intercalaires, est d'adapter la taille et la forme des prothèses à la grande diversité des situations anatomiques rencontrées dans la chirurgie de l'épaule. En théorie ce problème peut être résolu par un grand nombre de prothèses différentes et, éventuellement, de nombreux intercalaires, mais on comprend que de telles solutions deviennent rapidement coûteuses. De plus les problèmes d'approvisionnement et de maintenance dans les hôpitaux et dans les cliniques croissent très rapidement avec le nombre de prothèses de taille ou de forme différentes. Il en résulte que les solutions actuellement mises en oeuvre sont loin d'être optimales.

La présente invention se propose d'améliorer cette situation et d'assurer une meilleure adaptation des prothèses de tête humérale aux différentes situations anatomiques que l'on rencontre dans les têtes humérales, tout en maintenant le nombre de prothèses d'un jeu de prothèses à une valeur réduite.

Un autre avantage de l'invention est de simplifier la forme des prothèses d'un jeu et donc d'en diminuer le coût de fabrication.

L'invention a pour objet un jeu de prothèses de tête humérale destiné à coopérer avec un jeu de queues de prothèse humérale possédant une surface de réception (9) inclinée par rapport à l'axe longitudinal de la queue, lesdites prothèses de tête humérale comprenant une calotte sphérique, une base inférieure (3) susceptible d'être appliquée sur lesdites queues, et, s'étendant de ladite base inférieure (3), des moyens de fixation dans une position indexée sur les queues de prothèse humérale par un dispositif à téton susceptible d'être reçu et immobilisé dans un logement (10) correspondant de la queue sans présence d'une pièce intermédiaire entre la tête de prothèse et la queue de prothèse, de sorte que la base (3) de prothèse de tête humérale soit voisine de la surface (9) de queue de prothèse dans les différentes positions angulaires de la tête de prothèse autour desdits moyens de fixation, caractérisé en ce que lesdits moyens de fixation sont excentrés par rapport à la calotte sphérique et comprennent un téton (4) excentré par rapport à l'axe de celle-ci, en ce que ladite calotte sphérique se raccorde, à sa périphérie, à ladite base inférieure (3), et en ce que toutes les prothèses d'un même jeu présentent un rayon de courbure identique de calotte sphérique tandis que les diamètres d'encombrement maximal des différentes prothèses d'un même jeu sont différents.

De préférence le jeu est constitué de quatre prothèses.

Dans une forme de réalisation préférée le rayon de courbure de la calotte sphérique est de préférence de l'ordre de 25 mm.

Les diamètres des quatre prothèses du jeu s'échelonnent, de préférence, de 40 à 50 mm environ et de façon particulièrement préférée respectivement de 40, 44, 46 et 50 mm.

Les hauteurs de calotte sont alors, pour un même rayon de courbure de 26 mm, respectivement de 14, 18, 20 et 24 mm.

Grâce à l'invention, il n'est pas nécessaire que des prothèses du jeu de prothèses comportent un col.

Le téton est excentré dans la base, et ceci, de préférence, en formant un entraxe de l'ordre de 5 mm, de préférence identique pour toutes les prothèses du jeu.

Afin d'assurer l'indexation angulaire, on prévoit, de préférence, des moyens d'indexation à trois positions angulairement décalées dans les deux sens de rotation, à savoir une position 0°, dans laquelle l'axe de la calotte sphérique se trouve dans le plan de symétrie de la queue de prothèse dans lequel se trouve également l'axe du téton, et des positions, de part et d'autre, de 45 et 90°. En d'autres termes le décalage, généralement en rétroversion, est soit nul, soit de 45° soit de 90°.

Ces moyens d'indexation peuvent avantageusement être constitués de simples trous, le trou choisi pour l'indexation étant destiné à recevoir un petit téton faisant saillie de la surface de réception de tête humérale de la queue de prothèse.

De préférence la zone de plus grand diamètre de la calotte sphérique se raccorde à la base par un bord arrondi, de façon à éviter la formation d'une arrête aiguëe à l'intersection géométrique de la calotte sphérique et du plan de la base.

Les prothèses du jeu de prothèses de tête humérale peuvent coopérer avec un jeu de queues de prothèse humérale de tailles différentes mais présentant une surface munie de moyens identiques pour recevoir la base de prothèse de tête humérale avec ses moyens de fixation et, s'il y a lieu, d'indexation. Ainsi la queue de prothèse peut avantageusement comporter un logement tronconique dans lequel peut venir se disposer puis s'immobiliser par liaison du type conemorse, la prothèse de tête humérale. Cette surface de la queue de prothèse présente également avantageusement un petit téton destiné à pénétrer dans le trou d'indexation angulaire correspondant de la prothèse de tête humérale.

De préférence la surface de réception de tête humérale de la queue de la prothèse est inclinée de 45° par rapport l'axe longitudinal de la queue, c'est-à-dire l'axe du canal médullaire huméral.

De façon avantageuse on peut associer au jeu de prothèses selon l'invention, un jeu de prothèses d'essai dont les rayons de courbure et le diamètre de base sont identiques aux prothèses du jeu de prothèses selon l'invention. Cette prothèse d'essai, réalisée de préférence en matière synthétique, est destinée à venir se fixer de façon temporaire sur la râpe qui est utilisée pour agrandir le canal médullaire et déterminer le logement osseux dans lequel sera scellé la queue de prothèse définitive. De façon avantageuse, dans le cas de prothèses décentrées, la prothèse d'essai présente des moyens d'indexation assurant une indexation identique à celle des prothèses du jeu de prothèses de tête humérale, permettant au chirurgien, après avoir choisi la prothèse d'essai ayant les dimensions convenables, de déterminer l'angle d'indexation définitif sous lequel il fixera la prothèse définitive sur la queue de prothèse.

De préférence la partie en forme de calotte de la prothèse d'essai est capable de tourner et d'être indexée dans les différentes positions angulaires requises autour d'un téton de fixation destiné à venir se fixer de façon temporaire dans un trou correspondant de la râpe, par exemple par encliquetage.

A cet effet ce téton peut être déplaçable axialement, à l'encontre d'un ressort, dans un évidement de la calotte sphérique de la prothèse d'essai, et posséder un bras muni d'un ergot susceptible de rentrer dans l'un des trous d'indexation de la base de la prothèse d'essai, d'où il peut ensuite être dégagé facilement.

De préférence les prothèses d'un même jeu peuvent être présentées dans des emballages individuels stériles, de préférence à l'intérieur d'un même conteneur ou emballage commun.

En variante les différentes prothèses d'un jeu peuvent être disposées sans être emballées individuellement, à l'intérieur d'un même emballage commun stérile.

Les prothèses d'essais sont de préférence présentées en un jeu distinct des prothèses formant le jeu de prothèses définitives.

Cependant les prothèses peuvent également, en variante, être livrées, sans être emballées de façon stérile, du fait que l'hôpital ou la clinique complétera les jeux au fur et à mesure que les différentes prothèses individuelles sont utilisées, et les stérilisera.

D'autres avantages et caractéristiques de l'invention apparaîtront à la lecture de la description suivante, faite à titre d'exemple non limitatif et se référant au dessin annexé, dans lequel :
- la figure 1 représente une vue de dessous d'une prothèse de tête humérale selon l'invention ;
- la figure 2 représente une vue en élévation de cette prothèse ;
- la figure 3 représente une vue de côté de cette prothèse ;
- la figure 4 représente des vues de côté des quatre prothèses d'épaule d'un jeu de prothèses selon l'invention ;
- les figures 5 et 6 représentent des prothèses d'un jeu selon l'invention en faisant apparaître les cotes variables de ces prothèses ;
- la figure 7 représente une vue en élévation d'une queue de prothèse selon l'invention ;
- la figure 8 représente une vue de dessus de cette prothèse ;
- la figure 9 représente une vue en coupe de la partie supérieure de cette queue de prothèse ;
- la figure 10 représente une vue en coupe d'une prothèse d'essai selon l'invention ;
- la figure 11 représente une vue de dessous d'une telle prothèse d'essai.

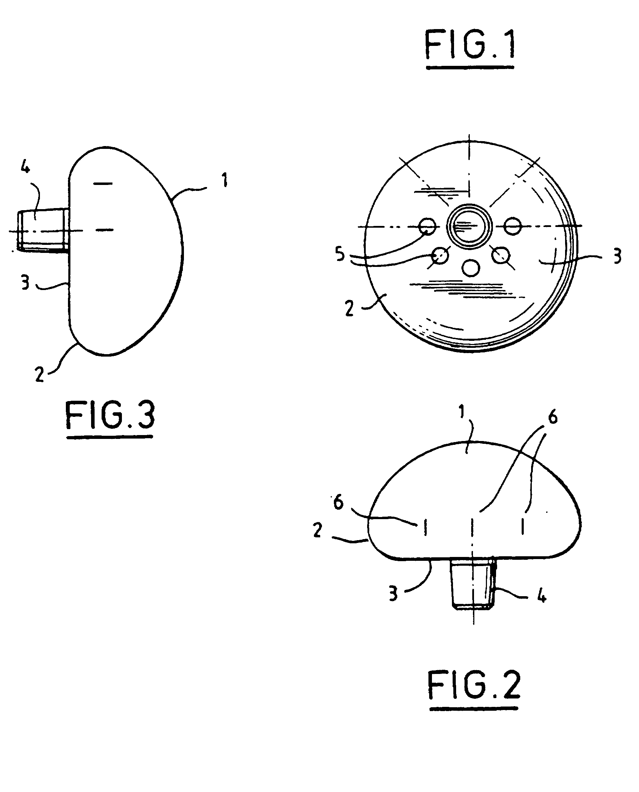

On se réfère tout d'abord aux figures 1 à 3.

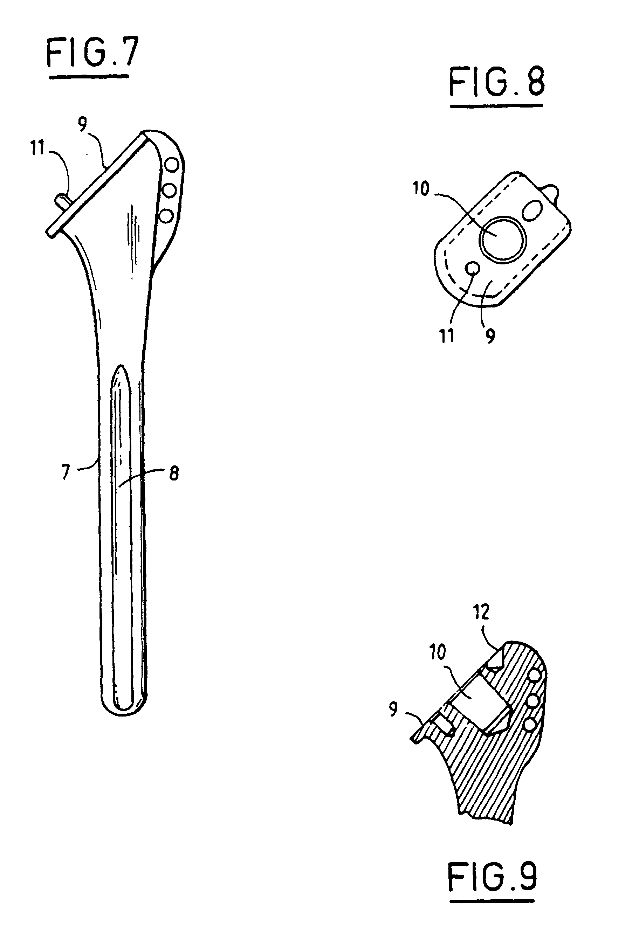

La prothèse complète de tête humérale selon l'invention est composée d'une queue de prothèse représentée sur les figures 7 à 9 et d'une prothèse de tête humérale proprement dite représentée plus précisément sur les figures 1 à 3. Cette prothèse, réalisée en titane, présente une forme de calotte sphérique 1 se raccordant, à sa périphérie, selon une zone arrondie 2, à une base inférieure plane 3. En une position excentrée par rapport à l'axe de révolution de la calotte sphérique, s'étend un téton de fixation tronconique 4 destiné à permettre la fixation et le blocage par un effet de cône morse de la prothèse de tête humérale sur la queue de prothèse. Autour du téton 4 se trouvent espacés, avec un intervalle angulaire de 45°, cinq trous 5, à savoir un trou central aligné dans le plan médian rejoignant l'axe du téton 4 à l'axe géométrique de révolution de prothèse et, en sens horaire, comme en sens antihoraire, deux trous disposés l'un à 45° du trou central et le second à 90°. Dans la zone de plus grand diamètre de la calotte sphérique 1, se trouvent disposés des marquages 6 effectués par laser, en correspondance angulaire (par rapport à l'axe du téton 4) des trous 5 et portant les marquages 0 pour le trou central et, 45° et 90° pour les autres trous.

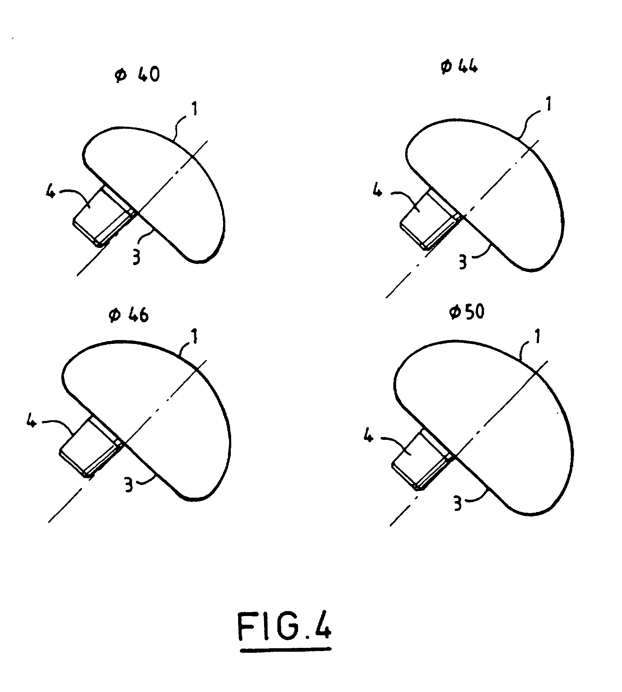

Le jeu de prothèses selon l'invention est constitué de quatre prothèses telles que représentées sur la figure 4 et ayant toutes le même rayon de courbure de calotte sphérique égales à 26 mm. Les diamètres des quatres têtes sont respectivement de 40, 44, 46 et 50 mm. Ces diamètres sont pris dans le plan d'encombrement maximal de la calotte sphérique.

Dans ces conditions les hauteurs, c'est-à-dire les distances entre la base 3 et le sommet de la calotte sphérique sont, de préférence, respectivement de 19, 23, 25, 28 mm. La distance entre l'axe géométrique du téton 4 et l'axe de révolution de la calotte sphérique, c'est-à-dire l'excentration du téton est constante pour chaque tête de prothèse, et égale à 5 mm.

En se référant plus particulièrement aux figures 8 et 9, on voit une queue de prothèse 7 présentant la queue proprement dite allongée 8 et une partie supérieure évasée et incurvée en fonction du débouché qui sera pratiqué dans le canal médullaire de la partie supérieure de l'humérus grâce à une râpe de forme correspondante. Cette queue est de forme classique et présente, à son extrémité supérieure, une surface 9, inclinée de 45° par rapport à l'axe longitudinal de la queue. Dans cette surface est pratiqué un trou conique 10 destiné à recevoir le téton 4 et à l'immobiliser. Dans le plan médian et à une certaine distance on trouve un petit ergot ou doigt 11 qui a été inséré dans un trou correspondant que l'on voit sur la figure 9. Ce téton 11 est destiné à pénétrer dans l'un des trous 5 en fonction de la position angulaire qui aura été choisie par le chirurgien. La surface 9 présente encore un trou 12 parallèle à l'axe longitudinal de la queue de prothèse et donc incliné de 45° par rapport au plan de la surface 9, ce trou étant destiné à la fixation amovible d'un ancillaire de pose.

Pour la pose de la prothèse selon l'invention le chirurgien, après avoir pratiqué une voie d'abord convenable, par exemple, delto-pectorale, ressèque d'abord la tête humérale déficiente selon un plan de coupe inclinée de 45° par rapport à l'humérus et orienté en rotation autour de l'axe de la diaphyse, en fonction de la position anatomique qu'il désire obtenir pour la tête humérale. Il procède ensuite à la préparation du canal modulaire en utilisant une série de râpes de dimensions croissantes dont la dernière correspond aux dimensions de la queue de prothèse qu'il souhaite mettre en place.

Après avoir scellé la queue de prothèse en place il fixera, après avoir procédé à un essai à l'aide d'une prothèse d'essai, une prothèse de tête humérale prise dans le jeu de quatre prothèses, selon l'angle d'antéversion ou plus généralement de rétroversion, déterminé à l'aide de la prothèse d'essai. La prothèse est immobilisée angulairement lorsque le téton 11 pénètre dans le trou 5 correspondant à l'angle choisi.

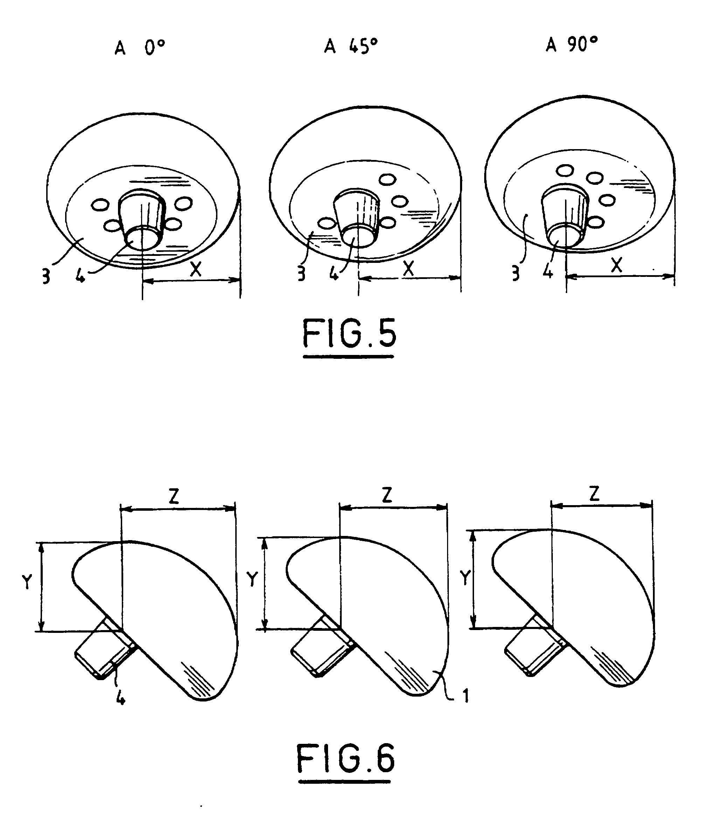

Une tête de prothèse donnée présente, une fois posée, les caractéristiques générales qui apparaissent sur les figures 5 et 6. Sur ces figures la distance X représente, en fonction de l'angle d'indexation choisi pour la prothèse, c'est-à-dire du trou 5 qui coopérera avec le téton 11, l'encombrement maximum dans le plan rejoignant l'axe de géométrie du téton 4 à l'axe du trou d'indexation 5 correspondant. La distance Y représente, pour chacune des trois positions l'ascensionnement, c'est-à-dire la distance du point le plus haut de la prothèse posée à un point de référence déterminé comme étant l'intersection de l'axe géométrique du téton 4 et du plan de la base 3. La distance Z représente la latéralisation, c'est-à-dire l'encombrement horizontal en direction de la glène lorsque la prothèse est posée.

Les valeurs X, Y et Z apparaissent sur le tableau I pour chaque valeur d'indexation, c'est-à-dire 0, 45 et 90°, et ceci pour chacune des têtes définis par son diamètre. On voit que, malgré des orientations très différentes, l'ascensionnement Z varie au maximum d'une valeur faible de 3,5 mm, ce qui présente un avantage considérable puisqu'il laisse subsister l'espace désirable entre la tête humérale et les parties anatomiques sus-jacentes et notamment la coiffe.

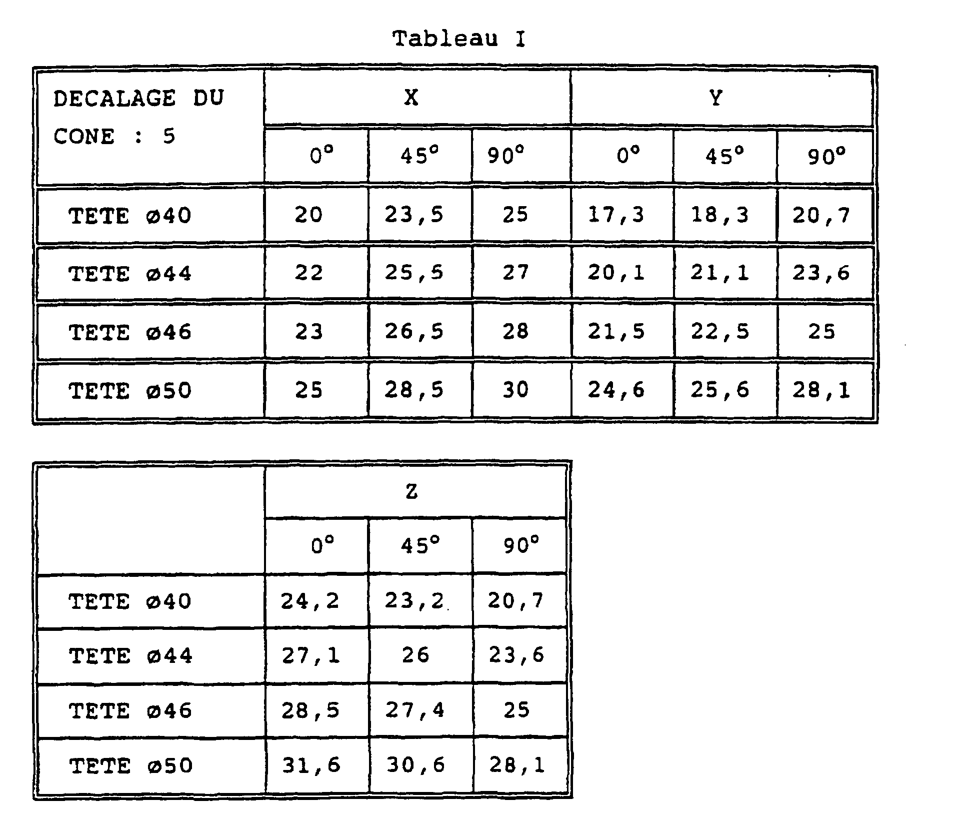

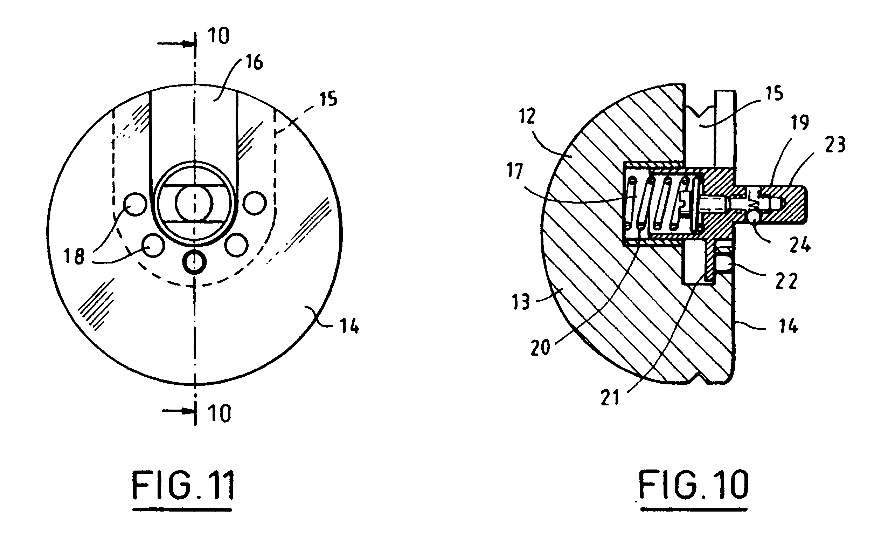

Pour choisir le diamètre de la prothèse à poser, ainsi que l'angle d'indexation de la prothèse, on peut avantageusement utiliser une tête d'essai telle que représentée sur les figures 10 et 11. De préférence il est prévu également quatre prothèses d'essai. La prothèse d'essai 12 peut être réalisée en un matériau tel qu'une matière synthétique stérilisable puisqu'elle n'est pas destinée à rester en place. Elle représente une forme de calotte sphérique 13 avec une base 14. Dans un plan proche de la base se trouve une entaille radiale 15 ouverte, par une fente de plus faible largeur 16 sur la base 14. Un trou borgne cylindrique 17 est pratiqué dans la calotte selon un axe géométrique excentré par rapport à l'axe de révolution de la calotte, d'une distance identique à l'excentration des prothèses, c'est-à-dire 5 mm. Cinq trous 18 sont pratiqués dans la base 14 avec des intervalles angulaires correspondant exactement aux intervalles angulaires séparant les trous d'indexation 5. Ces trous débouchent dans l'évidement 15.

Un téton réglable 19 est monté pivotant dans le trou 17 en étant repoussé vers la base 14 par un ressort 20. Ce téton 19 présente un bras latéral 21 se terminant par un ergot d'indexation 22. Par ailleurs le téton 19 présente, comme on le voit bien sur la figure 11, un méplat 22 perpendiculaire au bras 21. Un bille d'encliquetage provisoire 24 est repoussée légèrement hors d'une des faces latérales du téton 2 par un moyen élastique convenable.

L'utilisation de la tête d'essai s'effectue lorsque le chirurgien a fini d'utiliser la râpe de plus grande taille dont les dimensions correspondent à la queue de prothèse qui sera choisie. A ce moment la râpe de plus grande taille se trouve exactement dans la position qui sera occupée par la queue de prothèse définitive et sa surface supérieure se trouvera donc très précisément dans la position qui sera occupée par la surface supérieure 9 de la queue de prothèse. Le chirurgien choisit alors la tête d'essai de diamètre convenable et la place sur la surface de la râpe en faisant pénétrer le téton 18 dans le trou correspondant. Le téton 19, par son méplat, se trouve angulairement fixe par rapport à la râpe. Le chirurgien choisit l'angle d'indexation voulu en faisant tourner la calotte sphérique 13 par rapport au téton 19 jusqu'à introduire le doigt 22 dans le trou convenable. Après avoir ainsi déterminé et retenu le diamètre de prothèse et l'angle d'indexation de prothèse il retire la tête d'essai puis la râpe, met en place de façon définitive la queue de prothèse puis place, dans la position angulaire souhaitée, la tête de diamètre retenue.

La tête d'essai pourrait en variante, être utilisée sur la queue de prothèse déjà scellée.

L'invention permet, avec un jeu de prothèses réduit à quatre tailles, de couvrir avec une adaptation très fine à chaque cas particulier, l'ensemble des variations dimensionnelles tant en diamètre qu'en angulation en ayant, dans chaque cas, une latéralisation et un ascensionnement (X, Y, Z) bien adaptés.

Bien entendu l'invention peut faire l'objet de nombreuses variantes et, notamment, les moyens d'indexation peuvent différés.

## Revendications

1. Jeu de prothèses de tête humérale destiné à coopérer avec un jeu de queues de prothèse humérale possédant une surface de réception (9) inclinée par rapport à l'axe longitudinal de la queue, lesdites prothèses de tête humérale comprenant une calotte sphérique, une base inférieure (3) susceptible d'être appliquée sur lesdites queues, et, s'étendant de ladite base inférieure (3), des moyens de fixation dans une position indexée sur les queues de prothèse humérale par un dispositif à téton susceptible d'être reçu et immobilisé dans un logement (10) correspondant de la queue sans présence d'une pièce intermédiaire entre la tête de prothèse et la queue de prothèse, de sorte que la base (3) de prothèse de tête humérale soit voisine de la surface (9) de queue de prothèse dans les différentes positions angulaires de la tête de prothèse autour desdits moyens de fixation, **caractérisé en ce que** lesdits moyens de fixation sont excentrés par rapport à la calotte sphérique et comprennent un téton (4) excentré par rapport à l'axe de celle-ci, **en ce que** ladite calotte sphérique se raccorde, à sa périphérie, à ladite base inférieure (3), et **en ce que** toutes les prothèses d'un même jeu présentent un rayon de courbure identique de calotte sphérique tandis que les diamètres d'encombrement maximal des différentes prothèses d'un même jeu sont différents.

2. Jeu de prothèses selon la revendication 1, **caractérisé en ce que** le rayon de courbure de la calotte sphérique est de l'ordre de 25 mm.

3. Jeu de prothèses selon l'une des revendications 1 et 2, **caractérisé en ce qu'**il est constitué de quatre prothèses.

4. Jeu de prothèses selon la revendication 3, **caractérisée en ce que** les diamètres des quatre prothèses du jeu s'échelonnent de 40 à 50 mm environ et notamment respectivement de 40, 44, 46 et 50 mm.

5. Jeu de prothèses selon la revendication 4, **caractérisé en ce que** les hauteurs de calotte sont, pour un même rayon de courbure de 26 mm, respectivement de 14, 18, 20 et 24 mm.

6. Jeu de prothèses selon l'une des revendi-5 cations 1 à 5, **caractérisé en ce que** les prothèses du jeu de prothèses ne comportent pas de col.

7. Jeu de prothèses selon l'une des revendications 1 à 6, **caractérisés en ce que** la calotte sphérique (1) se raccorde à la base (3) par un bord arrondi (2).

8. Jeu de prothèses selon l'une des revendications 1 à 7, **caractérisé par** un entraxe identique pour toutes les prothèses du jeu.

9. Jeu de prothèses selon la revendication 8, **caractérisé par** un entraxe de 5 mm.

10. Jeu de prothèses selon l'une des revendications 1 à 9, **caractérisé par** des moyens d'indexation à trois positions angulairement décalées dans les deux sens de rotation.

11. Jeu de prothèses selon la revendication 10, **caractérisé en ce que** lesdits moyens d'indexation sont disposés en trois positions, à savoir une position 0°, dans laquelle l'axe de la calotte sphérique se trouve dans le plan de symétrie de la queue de prothèse dans lequel se trouve également l'axe du téton, et des positions, de part et d'autre, de 45 et 90°.

12. Jeu de prothèses selon l'une des revendications 10 et 11, **caractérisé en ce que** ces moyens d'indexation sont constitués de simples trous (15), le trou choisi pour l'indexation étant destiné à recevoir un petit téton (11) faisant saillie d'une surface (9) de réception de tête humérale de la queue de prothèse.

13. Jeu de prothèses d'essai munies de moyens de fixation provisoires et destinées au choix, pendant une opération de pose de prothèse, d'une tête de prothèse humérale selon l'une des revendications 1 à 12, dont les rayons de courbure et le diamètre d'encombrement maximum sont identiques aux prothèses du jeu de prothèses selon l'une des revendications 1 à 12 et les moyens de fixation provisoires sont excentrés de façon identique.

14. Prothèses d'essai selon la revendication 13, à téton excentré **caractérisées en ce que** la prothèse d'essai présente des moyens d'indexation (18, 21) assurant une indexation identique à celle des prothèses du jeu de prothèses de tête humérale.

15. Prothèses d'essai selon la revendication 14, **caractérisées en ce que** la partie en forme de calotte de la prothèse d'essai est capable de tourner et d'être indexée dans les différentes positions angulaires requises autour d'un téton de fixation (19) destiné à venir se fixer de façon temporaire dans un trou correspondant d'une râpe.

16. Prothèses d'essai selon l'une des revendications 14 et 15, **caractérisées en ce qu'**un téton est déplaçable axialement, à l'encontre d'un ressort, dans un évidement (15) de la calotte sphérique de la prothèse d'essai, et possède un bras 21 muni d'un ergot 22 susceptible de rentrer dans l'un des trous d'indexation de la base (14) de la prothèse d'essai, d'où il peut ensuite être dégagé facilement.

## Patentansprüche

1. Satz von Humeruskopf-Prothesen, der bestimmt ist mit einem Satz von Humerus-Prothesenstielen zusammenzuwirken, welche eine Aufnahmefläche (9) haben, welche bezüglich der Längsachse des Stieles geneigt ist, wobei die Humeruskopf-Prothesen eine sphärische Kappe, eine untere Basis (3), welche auf den Stielen befestigbar ist, und Fixierungsmittel aufweisen, die sich von der unteren Basis (3) aus erstrecken und die in einer Indizierungs-Position auf den Humerus-Prothesenstielen mittels einer Ansatz-Vorrichtung sind, die in einem korrespondierenden Sitz (10) des Stiels aufnehmbar und immobilisierbar ist ohne die Anwesenheit eines Zwischenstücks zwischen dem Prothesen-Kopf und dem Prothesenstiel, derart, dass die Basis (3) der Humeruskopf-Prothese der Fläche (9) des Prothesenstiels in den verschiedenen Winkel-Positionen des Prothesen-Kopfes um die Befestigungsmittel herum benachbart ist, **dadurch gekennzeichnet, dass** die Befestigungsmittel bezüglich der sphärischen Kappe exzentrisch sind und einen bezüglich der Achse von dieser exzentrisch angeordneten Ansatz (4) aufweisen, dass sich die sphärische Kappe mit ihrem Umfang an die untere Basis (3) anschließt, und dass bei allen Prothesen eines gleichen Satzes der Krümmungsradius der sphärischen Kappe identisch ist, wohingegen die maximalen Einhülldurchmesser der unterschiedlichen Prothesen eines gleichen Satzes unterschiedlich sind.

2. Prothesen-Satz gemäß Anspruch 1, **dadurch gekennzeichnet, dass** der Krümmungsradius der sphärischen Kappe im Bereich von 25 mm ist.

3. Prothesen-Satz gemäß Anspruch 1 oder 2, **dadurch gekennzeichnet, dass** er sich aus vier Prothesen zusammensetzt.

4. Prothesen-Satz gemäß Anspruche 3, **dadurch gekennzeichnet, dass** die Durchmesser der vier Prothesen des Satzes sich von ungefähr 40 bis 50 mm und insbesondere jeweils zu 40, 44, 46 und 50 mm staffeln.

5. Prothesen-Satz gemäß Anspruch 4, **dadurch gekennzeichnet, dass** für einen gleichen Krümmungsradius von 26 mm die Höhe der Kappen 14, 18, 20 bzw. 24 mm ist.

6. Prothesen-Satz gemäß einem der Ansprüche 1 bis 5, **dadurch gekennzeichnet, dass** die Prothesen des Prothesen-Satzes keinen Kragen aufweisen.

7. Prothesen-Satz gemäß einem der Ansprüche 1 bis 6, **dadurch gekennzeichnet, dass** die sphärische Kappe (1) sich an der Basis (3) mit einem runden Rand (2) anschließt.

8. Prothesen-Satz gemäß einem der Ansprüche 1 bis 7, **gekennzeichnet durch** einen identischen Achsabstand für alle Prothesen des Satzes.

9. Prothesen-Satz gemäß Anspruch 8, **gekennzeichnet durch** einen Achsabstand von 5 mm.

10. Prothesen-Satz gemäß einem der Ansprüche 1 bis 9, **gekennzeichnet durch** Indizierungsmittel an drei Positionen, welche in beiden Drehrichtungen winkelweise versetzt sind.

11. Prothesen-Satz gemäß Anspruch 10, **dadurch gekennzeichnet, dass** die Indizierungsmittel an drei Positionen angeordnet sind, nämlich einer 0°-Position, in welcher sich die Achse der sphärischen Kappe in der Symmetrieebene des Prothesen-Stiels befindet, in welcher sich auch die Achse des Ansatzes befindet, und Positionen beiderseits bei 45° und 90°.

12. Prothesen-Satz gemäß Anspruch 10 oder 11, **dadurch gekennzeichnet, dass** die Indizierungsmittel von einfachen Löchern (15) gebildet sind, wobei das zur Indizierung ausgewählte Loch bestimmt ist, einen kleinen Ansatz (11) aufzunehmen, der von einer Humeruskopf-Aufnahmefläche (9) des Prothesen-Stiels hervorsteht.

13. Versuchsprothesen-Satz, versehen mit provisorischen Befestigungsmitteln, die, während einer Implantier-Operation der Prothese, für einen Humerus-Prothesenkopf gemäß einem der Ansprüche 1 bis 12 auswählbar sind, dessen Krümmungsradien und maximaler Einhülldurchmesser mit den Prothesen des Prothesensatzes gemäß einem der Ansprüche 1 bis 12 identisch sind, wobei die provisorischen Befestigungsmittel auf identische Art und Weise exzentrisch sind.

14. Versuchsprothesen gemäß Anspruch 13, mit exzentrischem Ansatz, **dadurch gekennzeichnet, dass** die Versuchsprothese Indizierungsmittel (18, 21) aufweist, die eine identische Indizierung für jene von den Prothesen des Satzes von Humeruskopf-Prothesen sicherstellt.

15. Versuchsprothesen gemäß Anspruch 14, **dadurch gekennzeichnet, dass** der kappenförmige Abschnitt der Versuchsprothese drehbar ist und in den unterschiedlichen, erforderlichen Winkelpositionen um einen Fixierungs-Ansatz (19) herum indizierbar ist, der temporär in einem Loch fixierbar ist, welches einer Raspel zugeordnet ist.

16. Versuchsprothesen gemäß Anspruch 14 oder 15, **dadurch gekennzeichnet, dass** ein Ansatz gegen eine Feder in einer Aussparung (15) der sphärischen Kappe der Versuchsprothese axial verlagerbar ist und einen Arm 21 hat, der mit einem Stift (22) versehen ist, der in eines der Indizierungs-Löcher der Basis (14) der Versuchsprothese einrückbar ist, von wo er einfach entkuppelt werden kann.

## Claims

1. Set of humeral head prostheses designed to cooperate with a set of humeral stem prostheses possessing a receiving surface (9) inclined with respect to the longitudinal axis of the stem, the said humeral head prostheses comprising a spherical cap, a lower base (3) capable of being applied to the said stems and, extending from the said lower base (3), means for fixing in an indexed position on the humeral stem prostheses by a device with a spigot capable of being received and immobilised in a corresponding housing (10) of the stem without the presence of an intermediate part between the prosthesis head and the prosthesis stem, so that the base (3) of the humeral head prosthesis is in proximity to the surface (9) of the prosthesis stem in different angular positions of the prosthesis head about the said fixing means, **characterised in that** the said fixing means are off-set with respect to the spherical cap and comprise a spigot (4) off-set with respect to the axis thereof, **in that** the said spherical cap is connected, at its periphery, to the said lower base (3), and **in that** all the prostheses of the same set have an identical radius of curvature to the spherical cap, while the maximum overall diameters of the different prostheses of the same set are different.

2. Set of prostheses according to claim 1, **characterised in that** the radius of curvature of the spherical cap is of the order of 25 mm.

3. Set of prostheses according to one of claims 1 and 2, **characterized in that** it consists of four prostheses.

4. Set of prostheses according to claim 3, **characterised in that** that the diameters of the four prostheses of the set range from approximately 40 to 50 mm and are in particular 40, 44, 46 and 50 mm respectively.

5. Set of prostheses according to claim 4, **characterized in that** the cap heights are 14, 18, 20 and 24 mm respectively, for a same radius of curvature of 26 mm.

6. Set of prostheses according to one of claims 1 to 5, **characterized in that** the prostheses of the set of prostheses do not have a neck.

7. Set of prostheses according to one of claims 1 to 6, **characterized in that** the spherical cap (1) is connected to the base (3) by a rounded edge (2).

8. Set of prostheses according to one of claims 1 to 7, **characterized by** an identical distance between axes for all the prostheses of the set.

9. Set of prostheses according to claim 8, **characterized by** distances between axes of 5 mm.

10. Set of prostheses according to one of claims 1 to 9, **characterized by** means for indexing in three positions angularly offset in both directions of rotation.

11. Set of prostheses according to claim 10, **characterized in that** the said indexing means are arranged in three positions, namely a 0° position, in which the axis of the spherical cap is located in the plane of symmetry of the prosthesis stem in which the axis of the spigot is also situated, and positions either side of 45° and 90°.

12. Set of prostheses according to one of claims 10 and 11, **characterized in that** the indexing means consist of plain holes (15) the hole chosen for indexing being designed to receive a small spigot (11) projecting from a surface (9) for receiving the humeral head of the stem of the prosthesis.

13. Set of test prostheses provided with provisional fixing means and intended for choosing, during an operation for positioning a prosthesis, a humeral prosthesis head according to one of claims 1 to 12, of which the radii of curvature and the maximum overall diameter are identical to the prostheses of the set of prostheses according to one of claims 1 to 12, and the provisional fixing means are offset in an identical manner.

14. Test prostheses according to claim 13, with an off-set spigot, **characterized in that** the test prosthesis has indexing means (18, 21), ensuring identical indexing to that of prostheses of the set of humeral head prostheses.

15. Test prostheses according to claim 14, **characterized in that** the part in the form of a cap of the test prosthesis is capable of turning and of being indexed in different required angular positions about a fixing spigot (19) designed to be fixed temporarily in a corresponding hole of a rasp.

16. Test prostheses according to one of claim 14 and 15, **characterized in that** a spigot can be moved axially, against a spring, in a recess (15) of the spherical cap of the test prosthesis, and possesses an arm 21 provided with a pin 22 capable of entering one of the indexing holes of the base (14) of the test prostheses, from which it can then be easily disengaged.
